Europäisches Patentamt

European Patent Office

Office européen des brevets

(11) Publication number: **0 310 913**
**A2**

## (12) EUROPEAN PATENT APPLICATION

(21) Application number: **88115866.1**

(22) Date of filing: **27.09.88**

(51) Int. Cl.⁴: **C12N 15/00** , //**C12R1:01**

(30) Priority: **30.09.87 US 103168**

(43) Date of publication of application:
**12.04.89 Bulletin 89/15**

(84) Designated Contracting States:
**AT BE CH DE ES FR GB GR IT LI LU NL SE**

(71) Applicant: **BIOTECHNICA DIAGNOSTICS INC**
**61 Moulton Street**
**Cambridge Massachusetts 02138(US)**

(72) Inventor: **French, Cynthia Kaye**
**28 Exeter Street Apt. 508**
**Boston Massachusetts 02116(US)**

(74) Representative: **Modiano, Guido et al**
**MODIANO, JOSIF, PISANTY & STAUB**
**Modiano & Associati Baaderstrasse 3**
**D-8000 München 5(DE)**

(54) **DNA isolation procedure.**

(57) A method for isolating high molecular weight double stranded DNA from cells characterized by high endogenous deoxyribonucleases, the method including breaking open the cells to release the double stranded DNA and deoxyribonucleases; contacting the released DNA and released deoxyribonucleases with an aqueous solution that includes (1) a protein denaturing agent effective to denature the deoxyribonucleases without denaturing the double stranded DNA to suppress the activity of the deoxyribonucleases, and (2) an agent capable of dissolving disulfide bonds to suppress the renaturation of the denatured deoxyribonucleases; and isolating the high molecular weight double stranded DNA from the aqueous solution.

EP 0 310 913 A2

# DNA ISOLATION PROCEDURE

## Background of the Invention

The invention relates to the isolation of DNA from cells.

Marmur, 3 J. Mol. Biol. 208 (1961), describes a procedure for isolating high molecular weight double stranded DNA from microorganisms. In general, the procedure includes disrupting the cells; removing the proteins by denaturation and centrifugation; removing the RNA by RNase degradation; and precipitating DNA with isopropanol. Marmur says (p. 208) that "[d]egradation by DNase and divalent metal ion contamination is prevented by the presence of chelating agents and by the action of sodium lauryl sulfate."

## Summary of the Invention

In general, the invention features, in one aspect, a method for isolating high molecular weight double stranded DNA (i.e., double stranded DNA having more than about 22 kilobases) from cells having high endogenous levels of deoxyribonucleases. The method includes breaking open the cells to release the double stranded DNA and the deoxyribonucleases; contacting the released DNA and deoxyribonucleases with an aqueous solution that includes (1) a protein denaturing agent effective to denature the deoxyribonucleases without denaturing the double stranded DNA to suppress the activity of the deoxyribonucleases and (2) an agent (e.g., betamercaptoethanol; dithiothreitol) capable of breaking disulfide bonds to suppress the renaturation of the denatured deoxyribonucleases; and isolating the high molecular weight double stranded DNA.

A high endogenous level of deoxyribonucleases is a level sufficiently high that, using standard high molecular weight DNA isolation procedures (e.g ., Marmur, supra; Coykendall et al., 30 Int. J. Syst. Bact. 559 (1980)), less than 50% of the DNA recovered is high molecular weight double stranded DNA.

The denaturing agent, when present in appropriate quantities (e.g., 4-6 M), denatures the high endogenous levels of the deoxyribonucleases at a sufficient rate so that at least 50% of the double stranded DNA recovered is high molecular weight DNA.

The preferred protein denaturing agents are chaotropic agents such as guanidine isothiocyanate and quanidine hydrochloride. Chaotropic agents disrupt the arrangement of water molecules around the hydrophillic regions of proteins to cause the proteins to denature.

Preferably, the cells are broken open in the aqueous solution so that the endogenous dexoyribonucleases present do not have time to substantially cleave the high moleclar weight DNA.

The invention features, in another aspect, a method for isolating high molecular weight double stranded DNA from cells, the method including breaking open the cells to release double stranded DNA and deoxyribonucleases; contacting the released DNA and released deoxyribonucleases with an aqueous solution that includes a chaotropic agent that denatures deoxyribonucleases without denaturing double stranded DNA to suppress the activity of the deoxyribonucleases, and an agent capable of breaking disulfide bonds to suppress the renaturation of denatured deoxyribonucleases; and isolating high molecular weight double stranded DNA from the aqueous solution.

The invention provides an easy, quick method for isolating intact double stranded DNA from cells, including those with high endogenous nuclease activity. The protein denaturing agents effectively denature the deoxyribonucleases while not denaturing the double stranded DNA ensuring that good yields of uncleaved (and thus high molecular weight), double stranded DNA can be isolated.

Other features and advantages of the invention will be apparent from the following description of the preferred embodiment thereof and from the claims.

## Description of the Preferred Embodiment

In the preferred method, cells containing high endogenous deoxyribonuclease activity are grown, and cells from the culture centrifuged to obtain a cell pellet containing intact cells. The cell pellet is then resuspended in an aqueous, buffered (pH 7.0-7.7) solution containing a detergent, an agent that dissolves disulfide bonds, a chaotropic agent, double stranded, and a chelating agent. High molecular weight DNA is subsequently isolated from the solution by standard CsCl gradient techniques.

The detergent disrupts the cell membranes so that the nucleic acids and proteins present in the cells are released. Many suitable detergents are known to those skilled in the art; a long list of biological detergents is supplied in the Sigma Chemical Co, catalogue. Generally the solution should contain between 0.5% and 5% detergent by weight. The concentration of detergent should be high enough so that the cell membranes are disrupted, but not so high that it causes the chaotropic agent to precipitate.

The chaotropic agent denatures the deoxyribonucleases (and other proteins) that are present in solution without disruptiong the hydrogen bonding between the DNA strands. The agents should be present in a high enough concentration so that proteins present in the solution are quickly denatured, but not so high that the solution cannot be spun properly with CsCl. Generally,·the chaotropic agent should be present in a concentration of 4.6 M. The preferred chaotropic agent is guanidine isothiocyante; other chaotropic agents, e.g., guanidine hydrochloride,· are well-known to those skilled in the art.

The agents that dissolve disulfide bonds break the disulfide bonds that are present in deoxyribonucleases to ensure that the enzymes do not renature into an active form. In general, even low concentrations (e.g., 10 mM) of the agent will have a positive effect, although the maximum effect of the agent is obtained at concentrations of approximately 100 mM; concentrations higher that 100 mM do not appreciably enhance the speed with which the agent acts. Suitable agents include betamercaptoethanol (most preferred) and dithiothreitol; many other agents are known to those skilled in the art.

The chelating agent, e g., EDTA, EGTA, assists in the denaturation of the deoxyribonucleases. Preferably, the concentration of the chelating agent is 15 mM or less; too much chelating agent may cause the chaotropic agent to precipitate. Effective denaturation of the enzymes occur even if a chelating agent is not present.

Suitable buffers include Tris-Cl buffers and phosphate buffers.

Intact DNA was isolated from Bacteroides intermedius as follows.

A 1 liter culture of Bacteroides intermedius was grown to log phase in mycoplasma broth base (BBL) supplemented with 0.5% glucose, 0.5 ug/ml hemin, and 0.3 ug/ml menadione at 37° C anerobically. The 0.D.600 was read, and the cells were centrifuged at 8,000 rpm for 10 minutes in a Sorvall RC-2B at 4° C. The cell pellet was resuspended by vortexing in five volumes of a solution of 5M guanidine isothiocyanate (BRL), 50 mM Tris-Cl pH 7.6, 10 mM EDTA, 2% s-lauryl sarcosinate (Sigma), and 140 mM betamercaptoethanol. One gram of CsCl was added to each 2.5 ml of homogenate. The homogenate was layered over a 2 ml cushion of 5.7 M CsCl in a 12 ml polyallomer centrifuge tube (Sorvall #03699) and the top overlayed with glycerol. The samples were spun at least 16 hours in the Sorvall RC70 ultracentrifuge at 35,000 rpm at 20° C in the swinging bucket TH641 rotor. After ultracentrifugation the contents of the tube were fractionated into 500 ul aliquots, and 5 ul from each aliquot was spotted on a 1% agarose plate containing 0.5 ug/ml ethidium bromide. The DNA containing fractions were visualized under ultraviolet light (Fotodyne), pooled, and dialyzed at 4° C against 10 mM Tris, 1 mM EDTA (TE) overnight.

A lug of double stranded DNA was electrophoretically analyzed. The DNA was intact, high molecular weight chromosomal DNA at a predominant size of 23 kilobases. The preparation appeared to be completely free of RNA. When subjected to restriction enzyme digestion, the fragments were of the appropriate size distribution for DNA cloning procedures.

The above procedure was repeated and similar results obtained on other bacteria having high endogenous deoxyribonuclease activity, including Bacteroides gingivalis, Actinobacillus actinomycetemcomitans, Wollinella recta, Eikenella corrodens, Fusobacterium nucleatum, Hemophilus - (several strains), and Candida albicans.


## Other Embodiments


Other embodiments are within the following claims. For example, the preferred aqueous solution can be used to isolate efficiently high molecular weight double stranded DNA from any cells, e.g., E. coli, regardless of the endogenous deoxyribonuclease activity of the cells.


## Claims

1. A method for isolating high molecular weight double stranded DNA from cells characterized by high endogenous levels of deoxyribonucleases, said method comprising

breaking open said cells to release said double stranded DNA and said deoxyribonucleases;

contacting said released DNA and released deoxyribonucleases with an aqueous solution comprising

a protein denaturing agent effective to denature said deoxyribonucleases without denaturing said double stranded DNA to suppress the activity of said deoxyribonucleases, and

an agent capable of breaking disulfide bonds thereby suppressing the renaturation of said denatured deoxyribonucleases; and

isolating said high molecular weight double stranded DNA from said aqueous solution.

2. The method of claim 1, wherein said protein denaturing agent comprises a chaotropic agent.

3. The method of claim 2, wherein said chaotropic agent is guanidine isothiocyanate.

4. The method of claim 1, wherein said agent that dissolves disulfide bonds comprises betamercaptoethanol.

5. The method of claim 1, wherein said cells comprise anaerobic bacteria.

6. The method of claim 5, wherein said bacteria comprise a member of the genus Bacteroides.

7. The method of claim 6, wherein said bacteria is a member of the species Bacteroides intermedius.

8. The method of claim 6 wherein said bacteria is a member of the species Bacteroides gingivalis.

9. The method of claim 1, wherein said contacting step and said breaking open step are carried out simultaneously.

10. A method for isolating high molecular weight double stranded DNA from cells containing said double stranded DNA and deoxyribonucleases, said method comprising
breaking open said cells to release said double stranded DNA and said deoxyribonucleases;
contacting said released DNA and released deoxyribonucleases with an aqueous solution comprising
a chaotropic agent for denaturing said deoxyribonucleases without denaturing double stranded said DNA to suppress the activity of said deoxyribonucleases, and
an agent capable of breaking disulfide bonds to suppress the renaturation of said denatured deoxyribonucleases; and
isolating said high molecular weight double stranded DNA from said aqueous solution.

11. The method of claim 10, wherein said chaotropic agent is guanidine isothiocyanate.

12. The method of claim 10, wherein said agent that dissolves disulfide bonds comprises betamercaptoethanol.